# EUROPEAN PATENT SPECIFICATION

(11) **EP 4 125 996 B1**
(45) Date of publication and mention of the grant of the patent: **28.08.2024**
(21) Application number: 21714634.9
(22) Date of filing: 31.03.2021
(51) Int. Cl.: A61K 38/17, A61P 31/14, C07K 14/47

(54) **EZRIN PEPTIDE 1 FOR USE IN A METHOD OF TREATING COVID-19**
EZRIN-PEPTID 1 ZUR VERWENDUNG IN EINEM VERFAHREN ZUR BEHANDLUNG VON COVID-19
PEPTIDE EZRINE 1 DESTINÉ À ÊTRE UTILISÉ DANS UN PROCÉDÉ DE TRAITEMENT DE LA COVID-19

(30) Priority: 01.04.2020 EP 20167623; 30.04.2020 EP 20172561; 11.05.2020 EP 20173939
(43) Date of publication of application: 08.02.2023
(73) Proprietor: Pantapharm AG, 37115 Duderstadt (DE)
(72) Inventor: NESSELHUT, Thomas, 37115 Duderstadt (DE); NESSELHUT, Jan, 37115 Duderstadt (DE); OSMERS, Rüdiger, 37115 Duderstadt (DE)
(74) Representative: Meissner Bolte Partnerschaft mbB
(86) International application number: PCT/EP2021/058459
(87) International publication number: WO 2021/198346

(56) References cited:
- WO-A1-2022/024108
- WO-A1-95/33768
- PETERS J HINRICH ET AL: "Akuttherapie von Viruskrankheiten", 24 March 2020 (2020-03-24), pages 1 - 3, XP055813111, Retrieved from the Internet <URL:https://jimdo-storage.global.ssl.fastly.net/file/79973013-b216-4ade-bc96-b1a4ecfb8a3b/Peters_Akuttherapie_von_Viruskrankheiten.pdf> [retrieved on 20210611]
- BESSLER WOLFGANG: "Arbeitsgruppe Vakzine", 24 March 2020 (2020-03-24), pages 1 - 23, XP055813145, Retrieved from the Internet <URL:http://web.archive.org/web/20200601141608/https://sites.google.com/site/alsanafreiburg/home/ak-vakzine> [retrieved on 20210611]
- HOLMS R. D. ET AL: "Review of Russian ezrin peptide treatment of acute viral respiratory disease and virus induced pneumonia; a potential treatment for covid-19", 12 April 2020 (2020-04-12), pages FP,1-41, XP055813400, Retrieved from the Internet <URL:https://www.researchgate.net/publication/340600041_Review_of_Russian_ezrin_peptide_treatment_of_acute_viral_respiratory_disease_and_virus_induced_pneumonia_a_potential_treatment_for_covid-19> [retrieved on 20210614], DOI: 10.13140/RG.2.2.10214.57925
- MILLET JEAN KAORU ET AL: "Ezrin Interacts with the SARS Coronavirus Spike Protein and Restrains Infection at the Entry Stage", PLOS ONE, vol. 7, no. 11, 21 November 2012 (2012-11-21), pages e49566, XP055813049, Retrieved from the Internet <URL:https://storage.googleapis.com/plos-corpus-prod/10.1371/journal.pone.0049566/1/pone.0049566.pdf?X-Goog-Algorithm=GOOG4-RSA-SHA256&X-Goog-Credential=wombat-sa@plos-prod.iam.gserviceaccount.com/20210611/auto/storage/goog4_request&X-Goog-Date=20210611T113929Z&X-Goog-Expires=86400&X-Goog-SignedHeaders=h> DOI: 10.1371/journal.pone.0049566
- ANONYMOUS: "Ezrin Peptide (HEP-1) for Treatment of Coronavirus Disease (COVID-19) Infection", 13 November 2020 (2020-11-13), pages 1 - 4, XP055813549, Retrieved from the Internet <URL:https://clinicaltrials.gov/ct2/show/NCT04627233> [retrieved on 20210614]
- VAN KAMPEN JEROEN J.A. ET AL: "Shedding of infectious virus in hospitalized patients with coronavirus disease-2019 (COVID19): duration and key determinants", MEDRXIV, 9 June 2020 (2020-06-09), XP055835315, Retrieved from the Internet <URL:https://www.medrxiv.org/content/10.1101/2020.06.08.20125310v1.full.pdf> [retrieved on 20210826], DOI: 10.1101/2020.06.08.20125310
- NALBANDIAN ANI ET AL: "Post-acute COVID-19 syndrome", NATURE MEDICINE, NATURE PUB. CO, NEW YORK, vol. 27, no. 4, 22 March 2021 (2021-03-22), pages 601 - 615, XP037424502, ISSN: 1078-8956, [retrieved on 20210322], DOI: 10.1038/S41591-021-01283-Z

## Description

The present invention concerns Ezrin peptide 1 for use in a method of treating COVID-19.

Coronavirus disease 2019 (COVID-19) is caused by a novel coronavirus called SARS-CoV-2. The acronym "SARS" stands for severe acute respiratory syndrome. An outbreak of COVID-19 in the Hubei province (China) at the end of 2019 is spreading globally and is impacting the health of people and of the economy worldwide. As of March 30, 2020, COVID-19 has been confirmed in about 750,000 people worldwide, carrying a mortality of approximately 3-5%, compared with a mortality rate of less than 1% from influenza. Most diagnosis relies on PCR using specimens from the respiratory tract.

On 11 March 2020, the Director-General of WHO declared the spate of infections caused by SARS-CoV-2 (COVID-19) a pandemic.

The novel coronavirus SARS-CoV-2 causes flu-like symptoms such as dry cough, fever, a runny nose and fatigue. There have also been reports of an itchy throat, headaches, joint pains, nausea, diarrhoea and shivering. The virus spreads from person to person. Droplet infection is the main mode of transmission. Transmission can take place directly, from person-to-person, or indirectly through contact between hands and the mucous membranes of the mouth, the nose or the conjunctiva of the eyes. There have been reports of persons who were infected by individuals who had only shown slight or non-specific symptoms of disease. The percentage of asymptomatic cases is unclear. It is currently thought that an infected person can go up to 14 days before noticing any symptoms. According to WHO, the incubation period is, on average, five to six days.

Among the cases reported to date, in three out of five cases infection was mild with symptoms, e.g. dry cough and fever less than 39.5°C, for about 1 week. This means that not all diseases that follow a SARS-CoV-2 infection take a serious course and require treatment. Unlike other acute infectious diseases progressing to sepsis, the severe courses of COVID-19 seemingly show prolonged progression from onset of first symptoms to life-threatening deterioration of (primarily) lung function including difficult breathing and pneumonia. Severe acute respiratory distress syndrome (ARDS) reflects the hallmark of a critical course of the disease. Treatment of the infection depends on the severity of the disease presentation (e.g. administering oxygen, maintaining fluid balance, if necessary administering antibiotics to combat bacterial co-infections) and also includes the treatment of relevant underlying chronic illnesses. Current management of COVID-19 is supportive and ARDS is the leading cause of mortality.

Accumulating evidence suggests that a subgroup of patients with severe COVID-19 might have a cytokine storm syndrome which leads to hyper-inflammation and irremediable lung tissue damage. It has been reported that a cytokine profile resembling sHLH (secondary haemophagocytic lymphohistiocytosis) is associated with COVID-19 disease severity, characterized by increased interleukin (IL)-2, IL-7, granulocyte-colony stimulating factor, interferon-γ inducible protein 10, monocyte chemo-attractant protein 1, macrophage inflammatory protein 1-α, and tumour necrosis factor-α. Predictors of fatality from a recent retrospective, multicentre study of 150 confirmed COVID-19 cases in Wuhan, China, included elevated ferritin (mean 1297.6 ng/ml in non-survivors vs 614.0 ng/ml in survivors; p<0.001) and IL-6 (p<0 0001), suggesting that mortality might be due to virally driven hyper-inflammation (Mehta et al., The Lancet, Vol. 395, p. 1033-1034 (March 2020)).

During the course of the COVID-19 pandemic, it has also become apparent that patients with COVID-19 suffer from late/long-term effects long after the acute infection has subsided. Although those affected have recovered from COVID-19 itself, they are by no means healthy. Instead, they suffer long-term effects such as chronic fatigue, breathing difficulties, such as shortness of breath, loss of smell, concentration difficulties, anxiety, and depression. These long-term symptoms are also termed post-COVID-19 syndrome, long COVID or late COVID.

There is an urgent need for an effective treatment of COVID-19. The current focus has been on the development of novel therapeutics, including anti-viral agents and vaccines. However, it may take at least several months, in the worst case more than 1 year, until a suitable new medication and a vaccine has been developed. Until then there is an urgent need for the prevention and/or treatment of ARDS, including hyper-inflammation, as a complication of COVID-19 using existing, approved therapies with proven safety profiles to address the immediate need to reduce the rising mortality.

The inventors have found out that Ezrin peptide 1 or a pharmaceutical composition comprising Ezrin peptide 1 and a pharmaceutically acceptable carrier is suitable for avoiding a critical course of the COVID-19 disease.

Ezrin protein, also known as cytovillin or villin-2, is a protein encoded in humans by the EZR gene. The peptide used for the present invention is ezrin peptide 1 and comprises or is a pharmaceutical tetradecapeptide NH2_Thr-Glu-Lys-Lys-Arg-Arg-Glu-Thr-Val-Glu-Arg-Glu-Lys-Glu_COOH (sequence ID No. 1: TEKKRRETVERKEKE), comprising 14 amino acid residues, which is known as HEP-1 peptide or human Ezrin peptide one (TEKKRRETVEREKE) and which was developed for the treatment of HIV-infection (WO 95/33768 A1). HEP-1 is known to have anti-viral hepatitis C biological activity and can be used for the treatment of the patients with hepatitis C (WO 2004/067024 A2). It has been additionally reported that HEP-1 has antiulcer biological activity and can be used for the treatment of ulcer diseases of the gastrointestinal tract (WO 2007/060440).

In a circular redacted by Peters and Bessler published 24-03-2020, it was noted that ezrin peptide 1 has activity against several viruses (retrieved from https:// jimdo-storage.global.ssl.fastly.net/file/79973013-b216-4ade-bc96-b1a4ecfb8a3b/ Peters_Akuttherapie_von_Viruskrankheiten.pdf).

The peptide used in the present invention, i.e. Ezrin peptide 1 can be synthesized by peptide synthetic chemistry well known in the art, e.g. from US 2016/0346383 and the references cited therein. For example, the peptides of the invention can be synthesized by liquid-phase synthesis using standard procedure or by solid-phase synthesis or by combinations thereof. When solid-phase synthesis is employed, then a solid phase is used, such as polystyrene resin or polyamide resin, or PEG hybrid polystyrene resin, or resin based on PEG. Different protective groups are used during the synthesis, for example, N-terminal protecting groups, t-Boc or FMOC protective groups. In some instances, fragments may be synthesized using solid-state methods and then coupled together in solution. Peptides can be synthesized from the carbonyl group side to amino group side of the amino acid chain in this method, although peptides are synthesized in the opposite direction in cells. In such methods, an amino-protected amino acid is bound to a substrate bead (i.e. a resin bead) forming a covalent bond between the carbonyl group and the resin. The amino group is then de-protected and reacted with the carbonyl group of the next amino-protected amino acid. The cycle is repeated as often as required in order to form the desired peptide chain. The synthesized peptide is then cleaved from the bead at the end of the procedure. The protecting groups for the amino groups mostly used in this peptide synthesis are 9-fluorenylmethyloxycarbonyl group ("Fmoc") and t-butyloxycarbonyl ("Boc"). The Fmoc group is removed from the amino terminus with base while the Boc group is removed with acid. Moreover, benzyloxycarbonyl (Z) groups or allyloxycarbonyl (Alloc) protective groups, or photo-removable (lithographic) protective groups, or side group protection technique may be employed. Peptide products are purified by HPLC separation or by any other purification method. Peptide structure is confirmed by amino acid analysis, mass spectrometry, and high performance liquid chromatography data.

"Subject" or "patient" as used herein refers to humans tested positive for SARS-CoV-2 or suspected of being infected with SARS-CoV-2. These subjects may be affected by Coronavirus disease 2019 (COVID-19) or are being suspected of developing Coronavirus disease 2019 (COVID-19). As mentioned above, COVID-19 is caused by a novel coronavirus called SARS-CoV-2.

COVID-19 as used herein is associated with one or more of the following symptoms: dry cough, fever, a runny nose, fatigue, itchy throat, headaches, joint pains, nausea, diarrhoea and shivering. In particular, COVID-19 is reported to be associated as main symptoms with dry cough, breathing symptoms, fever and fatigue.

It is reported that the median disease duration of COVID-19 in a patient with mild symptoms is 1-2 weeks. Severe courses may include hospitalization and intensive care for about 3-6 weeks.

The pharmaceutical compositions used for the invention may comprise optionally other pharmaceutically active substances, such as anti-viral, anti-bacterial, analgesic and/or anti-inflammatory substances. In a preferred embodiment, the pharmaceutical composition may also contain immune-stimulatory agents. Examples for anti-viral substances are acyclovir, ribavirin, valaciclovir, oseltamivir, remdesivir or zanamivir. Examples for anti-bacterial substances are antibiotics including those of the classes aminoglycosides, ansamycins, carbacephmes, cephalosporins, glycopeptides, macrolides, penicillins, polypeptides, quinolones, sulfonamides, tetracyclines, glycycyclines, oxazolidinones, amphenicols, pleuromutilins, lincosamides, streptogramins, steroid antibacterials, cyclopeptides lipopeptides, and mixtures thereof.

The term "about" as used herein, means in quantitative terms plus or minus 5%, or in another embodiment plus or minus 10%, or in another embodiment plus or minus 15%, or in another embodiment plus or minus 20%. In particular, the term "about" as used herein, means in quantitative terms plus or minus 5% or the respective value given.

"Pharmaceutically acceptable" is meant to encompass any carrier, which does not interfere with the effectiveness of the biological activity of the active ingredients and that is not toxic to the patient to whom it is administered. Examples of suitable pharmaceutical carriers are well known in the art and include phosphate buffered saline solutions, water, emulsions, such as oil/water emulsions, various types of wetting agents, sterile solutions etc.. Such carriers can be formulated by conventional methods and can be administered to the subject at an effective dose. Additional pharmaceutically compatible carriers can include gels, bioadsorbable matrix materials, implantation elements containing the therapeutic agent, or any other suitable vehicle, delivery or dispensing means or material(s).

The dosage of the pharmaceutical composition is determined by the physician on the basis of the patient-specific parameters, such as age, weight, sex, severity of the disease, *etc.* The terms "dosage regimen" or "mode of treatment" refer to a timely sequential or simultaneous administration of the Ezrin peptide 1 or its analogue or a combination thereof, and any other optional pharmaceutically active substance. This means that the components may be provided in a unit dosage form with physical contact to each other (e.g. one single tablet or solution) or as separate entities (e.g. two tablets or solutions) to be taken simultaneously or with a certain time difference. This time difference may be in the range of 0.5 hour and 1 day, preferably between 1 hour and 5 hours.

The dosage of Ezrin peptide 1 is not particularly critical, as Ezrin peptide 1 is not toxic even at high dosages of 50 mg per kg body weight per day or higher. Preferably, Ezrin peptide 1 is administered to an adult human in an amount of at least 0.1 mg per day, in particular at least 0.2 mg per day, e.g. in an amount of at least 0.5 mg per day, such as 0.1 to 50 mg per day or 0.2 to 20 mg per day or 0.5 to 10 mg per day. Typically for parental administrations lower dosages will be required than for oral or rectal administration. To achieve a rapid relief of the symptoms of COVID-19 it may be reasonable to administer at least 1 mg per day or at least 2 mg per day but in other cases a lower dosage may be suitable.

In one embodiment, the Ezrin peptide 1 is administered in a dosage in the range of or between about 0.2 and 8 milligrams (mg), preferably 1 to 5 mg, more preferably 2 to 4 mg per day. In another embodiment, the Ezrin peptide 1 is administered in a dosage in the range of 0.1 and 5 milligrams per day (mg/d), preferably 0.1 to 4 mg/d, more preferably 0.1 to 1 mg/d, especially 0.1 to 0.5 mg/d. It is also possible to start with higher dosage of e.g. in the range of 1 to 5 mg/d, more preferably 1 to 3 mg/d and then continue with lower dosage of e.g. 0.1 to 0.5 mg/d.

The Ezrin peptide 1 or the pharmaceutical composition comprising Ezrin peptide 1 may be administered to the subject prior to, during and after symptoms associated with COVID-19 are present. Preferably, it is administered immediately after the onset of at least one symptom associated with COVID-19. If this is not possible it should be preferably administered after a positive test result for SARS-CoV-2 infection has been received. If this is not possible it should be administered prior to or immediately after the onset of fever, cough and/or breathing problems. The Ezrin peptide 1 or the pharmaceutical composition comprising Ezrin peptide 1 should be preferably administered prior to the start of the hospitalization of the patient. As a general rule: the earlier, the better. The dosage may be administered in one portion daily or in several partial doses over the day or every second day. The administration should be made during the course of the infection and be maintained until the last of the symptoms associated with COVID-19 has disappeared for at least 1 day, better 2 days. The Ezrin peptide 1 or the pharmaceutical composition comprising Ezrin peptide 1 may also be administered prophylactically, e.g. before an infection may occur, for example 1 or 2 days before an infection may occur.

The pharmaceutical composition may be prepared and administered in any dosage form suitable for administration to the subject's body. It is formulated to be compatible with its intended route of administration. Examples of routes of administration include parenteral, e.g. intravenous, intradermal, intramuscular, intra-arterial or subcutaneous administration, and oral administration. Preferably, the administration route is subcutaneous.

When administered subcutaneously, the pharmaceutical composition is preferably injected into well perfused tissue, for example subumbellic tissue, for example subumbellically laterally at the height of the iliac spine.

The pharmaceutical composition may be formulated using any convenient adjuvant and/or physiologically acceptable diluents. The type of formulation depends on the route of administration in a known manner.

In a preferred embodiment, the formulation may be prepared as a tablet, a lozenge, a liquid, gel, a suspension, an emulsion or a solution.

Solutions or Suspensions used for parenteral, in particular subcutaneous, application can include the following components: a sterile diluent such as water for injection, (physiological) saline solution, phosphate buffered saline, fixed oils, polyethylene glycols, glycerine, propylene glycol, or other synthetic solvents; antibacterial agents such as benzyl alcohol or methyl parabens; antioxidants such as ascorbic acid or sodium bisulfate; chelating agents such as ethylenediamine tetraacetic acid (EDTA); buffers such as acetates, citrates, or phosphates and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for parenteral administration are injectables. For the preparation of injectables sterile aqueous solutions, dispersions or emulsions are used. Sterile powders for the extemporaneous preparation of sterile injectable solutions or dispersions may also be used. For parenteral administration, suitable carriers include physiological saline, bacteriostatic water, Cremophor^{®} EL (BASF, Parsippany, N.J.), or phosphate buffered saline (PBS). In all cases, the composition must be sterile and should be fluid to the extent that easy syringability exists. It should be stable under the conditions of manufacture and storage and must be preserved against the contaminating action of microorganism such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganism can be achieved by various antibacterial and antifungal agents, for example, parabens, chlorobutanol, phenol, ascorbic acid, thimerosal, and the like. According to embodiments, isotonic agents, for example, sugars, polyalcohols such as manitol, sorbitol, or sodium chloride in the composition are added. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by sterilization, e.g. by filtration or heat sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preparation is prepared by vacuum drying or freeze-drying, which yields a powder of the active ingredient plus any additional desired ingredient from a previously sterile-filtered solution thereof.

In a further preferred embodiment, the composition for use according to the present invention is suitable for oral administration. Oral compositions generally include inert diluents or edible carriers. For the purpose of oral therapeutic administration, the active compound can be incorporated into excipients and can be used in the form of tablets, troches, chewie, lozenges, gel caps, soft gel or capsules, e.g. gelatin capsules. Oral compositions can also be prepared using a fluid carrier for use as a mouthwash. Pharmaceutically compatible binding agents or adjuvant materials can be included as part of the composition. The tablets, pills, capsules, troches, lozenges and the like can contain any of the following ingredients, or compounds of a similar nature: a binder such as microcrystalline cellulose, gum tragacanth or gelatin; an excipient such as starch or lactose, a disintegrating agent such as alginic acid, primogel (sodium starch glycolate), or corn starch; a lubricant such as magnesium stearate; a glidant such as colloidal silicon dioxide; a sweetening agent such as sucrose or saccharin.

The composition for use according to the present invention may include any additional component as desired, as long as such components do not substantially erode the effectiveness of the Ezrin peptide 1. Of course, the additional components are typically pharmaceutically acceptable. Such components may include, for example, thickeners, sweeteners, flavorants, fragrances, additional pharmaceuticals, glycine, mannitol, silicone dioxide, silica gels, binders, vitamins, carriers, such as, for example, glycerin, starches, celluloses, chitins, water, alcohol, and minerals.

Fillers, carriers, preservatives, and stabilizers, which are usually used by persons skilled in drug delivery technology, may be used as an acceptable carrier or filler for preparation of the provided pharmaceutical compositions. For injections, distilled water or physiologic saline are predominantly used.

It was surprisingly found by the inventors that Ezrin peptide 1 has the surprising property to avoid a critical course of COVID-19 including the much-feared severe acute respiratory distress syndrome. SARS-CoV-2 is absolutely foreign to the human organism since it is a novel virus. Thus, the human body has no appropriate antibody response ready when the infection occurs. This means that following the infection a strong immune response in the patient occurs which may be accompanied by a high inflammatory reaction. This may result in a viral lung inflammation in the course of COVID-19 since immune cells migrate into the infected lung tissue and attack the infected lung tissue via cytotoxic immune cells. The lung tissue becomes more and more inflammatory due to the enormous high immunologic defense accompanied by the immigration of the cytotoxic immune cells. This makes the oxygen exchange more and more difficult and may irreversibly damage the lung tissue due to the development of lung fibrosis.

Ezrin peptide 1 is an immune-modulatory tetradecapetide which has shown an anti-viral effect in cell cultures (R. Ataullakhanov et al.; Antiviral Mechanisms of the drug 'Gepon': Modulation of Cytokine Gene Transcription in a J-96 Human Cell Line. Eksp Klin Gastroenterol. 2005;(1):14-9, 106). An explanation of the anti-viral effect was that the docking of the virus to the target cell could be prevented. In addition, it was shown in cell cultures that it has an effect on the inflammatory cytokine production and, thus, has an influence on the inflammatory stress. It reduces the production of pro- and inflammatory cytokins (e.g. IL-1, IL-6, TNF-alpha) but induces the synthesis of alpha- and beta interferons. The inventors concluded that due to the modification of the production of inflammatory cytokins it would be possible to modulate the immunologically induced inflammatory reaction which may occur in the course of COVID-19. Thus, the lung inflammation could be down-regulated or even be prevented. Due to the anti-inflammatory effect of Ezrin peptide 1 and its analogues, the risk of pulmonary fibrosis as a result of massive cytokine release in lung tissue can be significantly reduced or even be prevented.

Thus, the early administration of Ezrin peptide 1 after a SARS-CoV-2 infection is not only successful due to its anti-viral effect but also due to the anti-inflammatory effect for shortening the overall disease duration and preventing the invasion of the disease to the lung and taking a severe course, e.g. by developing ARDS. Even if the disease has already invaded the lung, the Ezrin peptide 1 administration is expected to prevent a progression of this severe complication of COVID-19.

### Examples

### Example 1: 69 year old patient,

risk factors: adipositas, AV block 1^{st} grade, hypertonus
Skiing trip to Switzerland (Grindelwald) from March 1^{st} - 7^{th}
   Course of Disease:
   08.03.2020: Loss of taste
   09.03.2020: Feeling ill with fatigue and headache
   11.03.2020: Same symptoms but fever over 38°C
   12.03.2020: Coronavirus testing; Same symptoms with fever to 39°C,
   13.03.2020: Positive test result; persisting symptoms with starting dry cough
   14.03.2020: 1^{st} Ezrin peptide 1 administration in the evening, persisting symptoms
   15.03.2020: 2^{nd} Ezrin peptide 1 administration in the morning, increasing symptoms
   16.03.2020: 3^{rd} Ezrin peptide 1 administration in the morning, increasing symptoms with strong eye pain
   17.03.2020: 4^{th} Ezrin peptide 1 administration in the morning, increasing symptoms with more cough
   18.03.2020: 5^{th} Ezrin peptide 1 administration in the morning, significant signs of recovery, no eye pain, decreasing fever
   19.03.2020: 6^{th} Ezrin peptide 1 administration in the morning, significant signs of recovery, normal body temperature, no fatigue
   20.03.2020: 7^{th} Ezrin peptide 1 administration in the morning, return of taste, good general condition
   21.03.2020: 8^{th} Ezrin peptide 1 administration in the morning, no signs of illness anymore
   Dosage: 2 mg Ezrin peptide 1, dissolved in saline solution, separated into 10 single doses (à 0.2 mg),
   1 single dose per subcutaneous administration daily

Surprisingly, the severity of symptoms after administration was significantly lower than one would have expected in a patient of this risk group. The course of the disease was also significantly shorter compared to the course of the disease expected for patients of this risk group. Severe complications such as fibrosis of the lungs did not occur.

### Example 2: 64 year old male patient,

Risk factors: hypertonus, asthma, COPD, hyperchlosterinaemia
Permanent medication:
   - Candesartan 8 mg
   - Foster (beclometason dipropionate, formoterol fumarate dihydrate)
   - Simvastatin
Dosage: Six single doses à 2 mg Ezrin peptide 1, dissolved in saline solution and administered subcutaneously (s.c.).
Course of Disease:
   - 23.04.2020: First occurrence of fever, 38.4°C;
   - 24.04.2020: Severe clinical symptoms with high fever above 39.0 °C, nausea, loss of taste, respiratory distress, dry cough, severe headache; Corona-PCR was prepared with positive result for SARS-CoV-2, First Ezrin peptide 1, 2 mg subcutaneous (s.c.) in the evening;
   - 25.04.2020: In the morning second dose of Ezrin peptide 1, 2 mg s.c.; body temperature dropped to 37.5°C in the morning, still general weakness, shortness of breath, cough, loss of appetite, in the evening body temperature again 39.0°C;
   - 26.04.2020: Third administration of Ezrin peptide 1, 2 mg s.c.; Still loss of appetite, slight cough, in the evening body temperature still 37.5°C;
   - 27.04.2020: Fourth dose of Ezrin peptide 1, 2mg s.c.; Slight cough, slight headache, body temperature 37.4°C;
   - 28.04.2020: Fifth administration of Ezrin peptide 1, 2 mg s.c.; Only slight pain of touching the scalp, still slight weakness, body temperature 37.3°C;
   - 30.04.2020: Sixth administration of Ezrin peptide 1, 2 mg s.c.; Significant improvement of clinical symptoms, normal temperature, macrohaematuria (inconspicuous urological consultation), CRP level >100 mg/I, a CT of the thorax was carried out because of suspected pneumonia;
   - 01. to 04.05.2020: antibiotic coverage due to increased CRP level, taste completely returned, no more cough, no more fever, clinical well-being.

### Example 3: 79 year old male patient,

Risk factor/pre-existing illnesses: COPD, Diabetes mellitus Typ2, 2 artificial heart valves, Apoplexia in 2018
Dosage: Single doses of 2 mg Ezrin peptide 1, dissolved in saline solution and administered subcutaneously (s.c.) on December 23, 2020 followed by 0.2 mg/d Ezrin peptide 1 (single doses, s.c.) for four days.
Course of Disease:
   - First symptoms: Beginning of December 2020: Cough, loss of sense of smell and taste;
   - Continuously worsening of cough, fatigue and loss of appetite, shortness of breath since 18.12.2020;
   - Positive Antigen-Test (Lateral-flow-test) 22.12.2020, positive PCR test for SARS-Cov2 on 23.12.2020;
   - 23.12.2020 begin of HEP-1 therapy with 2 mg/d for two days followed by 0.2 mg/d for four (4) days;
   - 25.12.2020 significant improvement of cough and general condition;
   - No further symptoms after completion of HEP-1 therapy.

### Example 4: 49 year old female patient,

Risk factor/pre-existing illnesses: Hypertension
Dosage: Single doses of 2 mg/d Ezrin peptide 1, dissolved in saline solution and administered subcutaneously (s.c.) for 2 days, followed by 0.2 mg/d Ezrin peptide 1 (single doses, s.c.) for four (4) days.
Course of Disease:
   - First symptoms: 19.12.2020 Severe cough, fatigue, chills;
   - Further symptoms: 21.12.2020 diarrhoea, thoracic pain, angina pectoris, shortness of breath;
   - Positive Antigen-Test (Lateral-flow-test) 21.12.2020, positive PCR test for SARS-Cov2 on 23.12.2020;
   - 23.12.2020 begin of HEP-1 therapy with 2 mg/d for two days followed by 0.2 mg/d for four days;
   - 25.12.2020 significant improvement of cough and general condition;
   - No further symptoms after completion of HEP-1 therapy.

### Example 5: 53 year old male patient,

Risk factor/pre-existing illnesses: Hypertension
Initial dosage: Single doses of 2 mg/d Ezrin peptide 1, dissolved in saline solution and administered subcutaneously (s.c.) for 2 days, followed by 0.2 mg/d Ezrin peptide 1 (single doses, s.c.) for four days.
Subsequent dosage: 0.2 mg/d Ezrin peptide 1 (single doses, s.c.) for fourteen (14) days.
Course of Disease:
   - First symptoms: 23.12.2020 Severe cough and rhinitis, limb pains, sore throat, loss of sense of smell and taste, angina pectoris, fatigue, chills;
   - 23.12.2020 begin of HEP-1 therapy with 2mg/d (single dose) for two days followed by 0,2mg/d for four days;
   - 26.12.2020 significant improvement of symptoms;
   - 28.12.2020: PCR-Re test for SARS-Cov2 still positive;
   - No further symptoms after completion of HEP-1 therapy until 13.01.2021, then severe shortness of breath;
   - 15.01.2021: Administration of 0,2 mg/d Ezrin peptide 1 (single dose) for fourteen (14) days;
   - No further shortness of breath after completion of HEP-1 therapy.

### Example 6: 68 year old female patient,

Risk factor/pre-existing illnesses: unknown
Dosage: Single doses of 0.2 mg/d Ezrin peptide 1, dissolved in saline solution and administered subcutaneously (s.c.) for ten (10) days.
Course of Disease:
   - First symptoms: 09.12.2020 fever, cough, headache, shortness of breath and fatigue;
   - Positive PCR test for SARS-Cov2 on 10.12.2020;
   - 10.12.2020 begin of HEP-1 therapy with 0.2 mg/d for ten (10) days;
   - 14.12.2020 significant improvement of symptoms;
   - 20.12.2020 complete recovery of symptoms.

## Claims

1. Ezrin peptide 1 for use in a method of treating COVID-19 in a subject, wherein the Ezrin peptide 1 has the amino acid sequence NH2_Thr-Glu-Lys-Lys-Arg-Arg-Glu-Thr-Val-Glu-Arg-Glu-Lys-Glu_COOH (SEQ ID NO: 1).

2. The Ezrin peptide 1 for the use of claim 1, wherein the Ezrin peptide 1 is dissolved in saline solution for subcutaneous administration.

3. The Ezrin peptide 1 for the use of claim 1 or claim 2, wherein the Ezrin peptide 1 is formulated for oral administration.

4. The Ezrin peptide 1 for the use of any one of claims 1 to 3, wherein it is administered daily.

5. The Ezrin peptide 1 for the use of any of claims 1 to 4, wherein the subject is a human tested positive for SARS-CoV-2 or suspected of being infected with SARS-CoV-2.

6. A pharmaceutical composition comprising Ezrin peptide 1 and a pharmaceutically acceptable carrier for use in a method of treating COVID-19, wherein the Ezrin peptide 1 has the amino acid sequence NH2_Thr-Glu-Lys-Lys-Arg-Arg-Glu-Thr-Val-Glu-Arg-Glu-Lys-Glu_COOH (SEQ ID NO:1).

7. The pharmaceutical composition of claim 6, which is formulated either for subcutaneous administration or oral administration.

## Patentansprüche

1. Ezrin-Peptid 1 zur Verwendung bei einem Verfahren zur Behandlung von COVID-19 bei einem Individuum, wobei das Ezrin-Peptid 1 die Aminosäuresequenz NH2_Thr-Glu-Lys-Lys-Arg-Arg-Glu-Thr-Val-Glu-Arg-Glu-Lys-Glu_COOH (SEQ ID NO:1) aufweist.

2. Ezrin-Peptid 1 zur Verwendung nach Anspruch 1, wobei das Ezrin-Peptid 1 in Salzlösung zur subkutanen Verabreichung gelöst ist.

3. Ezrin-Peptid 1 zur Verwendung nach Anspruch 1 oder Anspruch 2, wobei das Ezrin-Peptid 1 zur oralen Verabreichung formuliert ist.

4. Ezrin-Peptid 1 zur Verwendung nach einem der Ansprüche 1 bis 3, wobei es täglich verabreicht wird.

5. Ezrin-Peptid 1 zur Verwendung nach einem der Ansprüche 1 bis 4, wobei es sich bei dem Individuum um einen Menschen handelt, der positiv für SARS-CoV-2 getestet hat oder bei dem eine Infektion mit SARS-CoV-2 vermutet wird.

6. Pharmazeutische Zusammensetzung, umfassend Ezrin-Peptid 1 und einen pharmazeutisch unbedenklichen Träger, zur Verwendung bei einem Verfahren zur Behandlung von COVID-19, wobei das Ezrin-Peptid 1 die Aminosäuresequenz NH2_Thr-Glu-Lys-Lys-Arg-Arg-Glu-Thr-Val-Glu-Arg-Glu-Lys-Glu_COOH (SEQ ID NO:1) aufweist.

7. Pharmazeutische Zusammensetzung nach Anspruch 6, die entweder zur subkutanen oder zur oralen Verabreichung formuliert ist.

## Revendications

1. Peptide d'ezrine 1 destiné à être utilisé dans un procédé de traitement du COVID-19 chez un sujet, le peptide d'ezrine 1 ayant la séquence d'acides aminés NH2_Thr-Glu-Lys-Lys-Arg-Arg-Glu-Thr-Val-Glu-Arg-Glu-Lys-Glu_COOH (SEQ ID n° : 1).

2. Peptide d'ezrine 1 destiné à l'utilisation de la revendication 1, le peptide d'ezrine 1 étant dissout dans une solution saline destinée à une administration sous-cutanée.

3. Peptide d'ezrine 1 destiné à l'utilisation de la revendication 1 ou de la revendication 2, le peptide d'ezrine 1 étant formulé pour une administration orale.

4. Peptide d'ezrine 1 destiné à l'utilisation de l'une quelconque des revendications 1 à 3, dans lequel il est administré quotidiennement.

5. Peptide d'ezrine 1 destiné à l'utilisation de l'une quelconque des revendications 1 à 4, dans lequel le sujet est un être humain ayant été testé positif pour le SARS-CoV-2 ou étant suspecté d'être infecté par le SARS-CoV-2.

6. Composition pharmaceutique comprenant un peptide d'ezrine 1 et un véhicule, acceptable d'un point de vue pharmaceutique, destinée à être utilisée dans un procédé de traitement du COVID-19, le peptide d'ezrine 1 ayant la séquence d'acides aminés NH2_Thr-Glu-Lys-Lys-Arg-Arg-Glu-Thr-Val-Glu-Arg-Glu-Lys-Glu COOH (SEQ ID n° : 1).

7. Composition pharmaceutique de la revendication 6, qui est formulée soit pour une administration sous-cutanée, soit pour une administration orale.
